# FASCICULE DE BREVET EUROPEEN

(11) **EP 0 476 089 B1**
(45) Date de publication et mention de la délivrance du brevet: **08.11.1995**
(21) Numéro de dépôt: 91906303.2
(22) Date de dépôt: 28.03.1991
(51) Int. Cl.: A61B 1/12, A61M 1/00

(54) **DISPOSITIF POUR L'IRRIGATION ET LE DRAINAGE CONTINUS DE TISSUS OUCAVITES DE L'ORGANISME HUMAIN OU ANIMAL**
EINRICHTUNG ZUR BESPÜLUNG ODER KONTINUIERLICHEN DRAINIERUNG DES GEWEBES ODER KÖRPERHÖHLEN DES MENSCHLICHEN ODER TIERISCHEN ORGANISMUS
DEVICE FOR CONTINUOUSLY IRRIGATING AND DRAINING HUMAN OR ANIMAL BODY TISSUES OR CAVITIES

(30) Priorité: 04.04.1990 FR 9004339
(43) Date de publication de la demande: 25.03.1992
(73) Titulaire: Ferton Holding, CH-2800 Delemont (CH)
(72) Inventeur: FAVRE, Pierre, CH-1061 Villars-Mendraz (CH)
(74) Mandataire: Gauger, Hans-Peter, Dipl.-Ing.
(86) Numéro de dépôt international: CH9100081
(87) Numéro de publication internationale: WO9115149

(56) Documents cités:
- EP-A- 0 293 081
- WO-A-86/06964
- US-A- 3 900 022

## Description

La présente invention concerne un dispositif pour l'irrigation et le drainage continus de tissus ou cavités de l'organisme humain ou animal, au moyen d'un liquide physiologique d'irrigation, pendant une intervention du type endoscopique, ce dispositif comprenant au moins une pompe du type péristaltique, et des moyens pour contrôler ladite irrigation.

L'endoscopie est une technique qui se développe rapidement dans le milieu médical. Elle permet l'examen d'une cavité du corps humain grâce à des techniques d'observation utilisant des fibres optiques. Certaines de ces cavités doivent être dilatées pour permettre une exploration au moyen de l'endoscope. Il existe actuellement deux techniques pour obtenir cette dilatation : la technique utilisant un gaz par exemple de l'anhydride carbonique et celle qui utilise un liquide physiologique tel qu'une solution saline ou du glycocolle. L'utilisation de gaz est limitée à la chirurgie et tend à disparaître. Actuellement cette première technique n'est plus utilisée que pour effectuer un diagnostic utilisant une perfusion. La deuxième technique utilisant une injection de liquide est en revanche en forte progression. Elle permet non seulement l'observation mais également un rinçage et l'évacuation de débris. Cette technique est utilisée en chirurgie générale, en orthopédie, en urologie, en gynécologie, en oto-rhino-laryngologie, en endoscopie gastro-intestinale, en ophtalmologie, etc.

Dans ces différents secteurs, la circulation du liquide physiologique peut se faire en circuit ouvert ou en circuit fermé. Dans le cas où la circulation du fluide s'effectue en circuit fermé, il est nécessaire de maintenir une pression constante dans la cavité explorée. Dans le cas où la circulation du fluide se fait en circuit ouvert, il n'est pas nécessaire de maintenir une pression constante dans la cavité mais une pression sensiblement constante à la sortie de la buse de rinçage. La circulation de fluide en circuit fermé est utilisée principalement en chirurgie orthopédique, en urologie et en gynécologie où la dilatation de la cavité est nécessaire pour permettre les observations. La circulation de fluide en circuit ouvert est utilisée en chirurgie générale, en oto-rhino-laryngologie, pour un lavage gastrique et pour le lavage de plaies ouvertes.

Actuellement, la circulation de fluide en circuit fermé est obtenue par trois moyens connus qui sont :
- l'écoulement par gravité,
- l'écoulement forcé au moyen d'une pompe péristaltique,
- l'écoulement forcé et l'aspiration au moyen de deux pompes péristaltiques.

La première technique basée sur le principe de l'écoulement par gravité utilise une poche contenant du sérum physiologique, constitué par exemple par une solution saline à 0,9 % qui est suspendue à une hauteur déterminée définie par le chirurgien et qui est habituellement de l'ordre de 1 mètre au-dessus du patient. Cette poche est reliée à la zone d'intervention par une tubulure stérile qui est elle-même connectée à un instrument tel que par exemple un arthroscope introduit dans l'articulation, lorsque l'intervention est une arthroscopie. Une canule d'évacuation équipée d'une vanne est utilisée pour évacuer le liquide de manière hâtive ou continue, ce qui permet d'éliminer les débris engendrés au cours de l'intervention et d'assurer le renouvellement continu du sérum afin que le chirurgien puisse suivre visuellement son travail à l'intérieur de l'articulation.

La deuxième technique est sensiblement identique à la précédente, mais la pression engendrée dans la zone d'intervention n'est plus obtenue par une colonne d'eau mais par une pompe péristaltique. L'évacuation du liquide s'effectue comme précédemment par gravité au moyen d'une canule équipée d'une vanne ou d'un robinet permettant un réglage des débits.

La troisième technique utilise deux pompes péristaltiques dont l'une est disposée en amont de la zone d'intervention et l'autre en aval. La première est destinée à amener le liquide dans la zone d'intervention et la deuxième est destinée à assurer l'évacuation de ce liquide et des matières solides ou liquides qu'il transporte. Les deux pompes sont de préférence asservies et permettent d'assurer, du moins en théorie, une pression constante dans la zone d'intervention. Lorsque l'on effectue le diagnostic ou une chirurgie dans une cavité ouverte, les méthodes actuellement utilisées dérivent directement de la technique de distension par gravité ou de la technique utilisant une pompe péristaltique d'irrigation. L'évacuation du liquide peut également être effectuée au moyen d'une buse d'aspiration raccordée directement à une pompe à vide équipant le bloc opératoire.

Actuellement chaque type d'intervention nécessite un appareil spécifique. C'est ainsi que l'on utilise des pompes de la société américaine 3M ou des pompes de la société suisse ORTHOCONCEPT pour des interventions en arthroscopie, des systèmes de lavage commercialisés par la maison DAVOL pour l'irrigation de plaies ouvertes ou une pompe proposée par la société ORTHOCONCEPT pour des résections de prostate.

Le fait de devoir disposer d'une panoplie importante de systèmes spécifiques pour chacune des interventions prévues en salle d'opérations représente pour la clinique un investissement lourd qu'elle ne peut pas toujours assumer. A ce problème de coût peuvent s'ajouter le problème de stockage et les difficultés liées à la stérilisation, générateurs de confusion et de perte de temps.

La présente invention se propose de pallier les différents inconvénients mentionnés ci-dessus en mettant à disposition du chirurgien un appareil universel susceptible d'être utilisé quelle que soit l'intervention effectuée.

Dans ce but, le dispositif selon l'invention concerne l'irrigation et le drainage continus de tissus ou cavités de l'organisme humain ou animal comme il est décrit dans le document EP-A-0293081 et comprenant une première pompe péristaltique connectée à une tubulure pour fournir un liquide physiologique d'irrigation pendant une intervention endoscopique; un module permanent équipé d'un premier moteur à commande d'entraînement de la première pompe péristaltique; et au moins une cassette à usage unique amovible équipée de ladite tubulure.Pour un dispositif du type ledit l'invention est caractérisé en ce que le dispositif comporte des moyens d'identification sur le module permanent prévues pour être activés par des moyens programmés sur la cassette pour fournir des informations spécifiques au moteur à commande pour effectuer l'opération de la première pompe péristaltique selon une intervention endoscopique spécifique à effectuer.

Dans un mode de réalisation préféré, le dispositif comporte une deuxième pompe péristaltique connectée à une tubulure pour effectuer le drainage du liquide physiologique sur commande d'une deuxième moteur d'entraînement.

Selon une première variante, les deux pompes sont montées dans un module permanent.

Selon une seconde variante, les deux pompes sont montées dans une cassette, des axes d'entraînement de ces pompes étant montés sur le module permanent.

Dans ce cas, la cassette est équipée d'au moins quatre tubulures correspondant par paires à chacune desdites pompes péristaltiques, une première paire de tubulures dont l'une est couplée à un réservoir de liquide physiologique et dont l'autre est connectée à un instrument d'intervention sur le patient pour amener ledit liquide physiologique dans la zone d'intervention, et une deuxième paire de tubulures dont l'une est également connectée à un instrument d'intervention pour évacuer le liquide physiologique de la zone d'intervention et dont l'autre est couplée à un circuit d'évacuation.

La cassette peut comporter des moyens de codage spécifiques à chaque type d'intervention, et le module permanent peut comporter des moyens d'identification de ce code.

De préférence, les moyens de codage comportent au moins un organe de codage déposé à un emplacement déterminé en fonction de l'intervention prévue et les moyens d'identification comportent une série de détecteurs dont l'un au moins est activé par l'organe de codage de la cassette.

Cet organe de codage peut être un téton protubérant ménagé sur une face de la cassette.

D'une façon avantageuse, les détecteurs sont des interrupteurs activés mécaniquement, des détecteurs optiques, des détecteurs magnétiques ou des détecteurs capacitifs.

Selon une forme de réalisation avantageuse, le dispositif comporte des moyens de sécurité pour empêcher une réutilisation de la cassette à usage unique.

Ces moyens de sécurité peuvent comporter un téton solidaire de la cassette, agencé pour s'engager dans une rainure du module permanent et conçu pour se casser lors du retrait de la cassette.

Avantageusement, ces moyens de sécurité sont associés aux moyens d'identification.

La présente invention sera mieux comprise en référence à la description des exemples de réalisation et du dessin annexé dans lequel :
la figure 1 représente une vue en perspective d'une forme de réalisation avantageuse de l'appareil selon l'invention ,
la figure 2 représente une vue en perspective du module permanent du dispositif de la figure 1,
la figure 3 représente une vue en coupe longitudinale d'une cassette du dispositif selon l'invention,
la figure 4 représente une vue en coupe selon la ligne A-A' de la figure 3, de la cassette,
la figure 5 représente une vue en coupe selon la ligne B-B' de la cassette présentée par la figure 3, et
la figure 6 représente une vue de détail illustrant des moyens de verrouillage du dispositif.

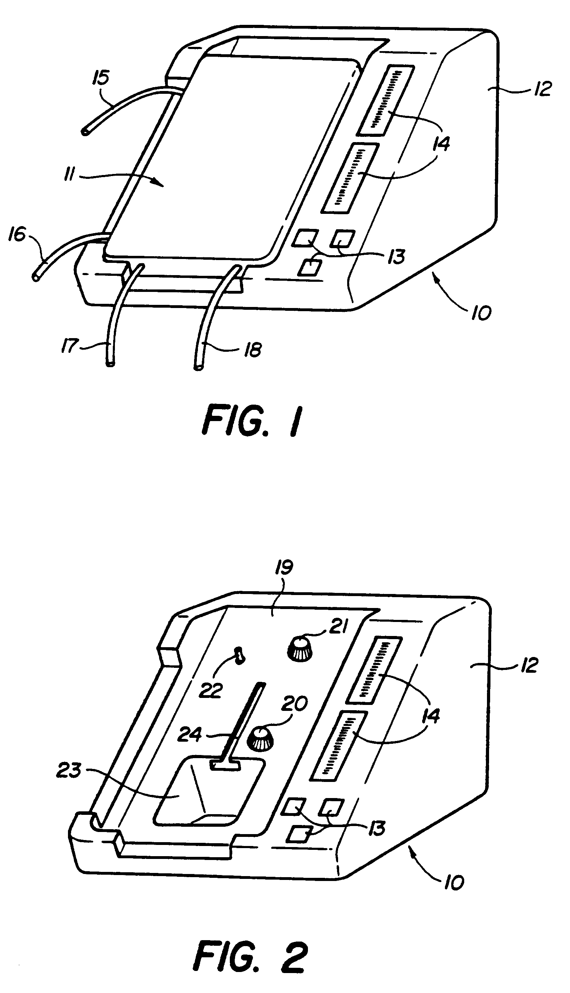

La figure 1 représente une forme de réalisation avantageuse du dispositif défini ci-dessus qui se compose essentiellement d'un module permanent 10 et d'une cassette amovible 11. Le module permanent comporte un boîtier 12 comprenant, par exemple sur sa face avant, des touches de fonction 13 et des indicateurs ou organes d'affichage 14 de grandeurs physiques telles que la pression, le débit, etc. Dans l'exemple représenté, la cassette 11 est équipée d'une tubulure d'approvisionnement 15 en liquide physiologique destinée à être raccordée à un réservoir contenant ce liquide (non représenté), une tubulure d'amenée 16 de ce liquide dans cette zone d'intervention du patient, une tubulure d'aspiration 17 destinée à aspirer le liquide physiologique dans cette zone et une tubulure d'évacuation 18 qui aboutit à un circuit d'évacuation où sont rejetés le liquide et les éventuels débris aspirés dans la zone d'intervention.

Comme mentionné précédemment, la cassette correspond à une application donnée et contient, d'une part deux pompes péristaltiques correspondant respectivement aux tubulures 15 et 16 et aux tubulures 17 et 18 et d'autre part, des moyens pour mémoriser les fonctions de ces pompes et notamment les paramètres de pression, de débit ou d'autres paramètres physiques qui doivent être contrôlés pour une intervention déterminée sur un patient.

La figure 2 présente une vue du module permanent lorsque la cassette a été retirée. Ce module permanent comporte un évidement 19 dans lequel peut être engagée une cassette et dans lequel apparaissent deux axes d'entraînement respectivement 20 et 21 des pompes péristaltiques (non représentées) logées dans la cassette 11 (voir figure 1). Au fond de cet évidement sont également disposés un embout de raccordement 22 destiné à la prise de pression, ainsi qu'une cavité 23 et une rainure 24 communiquant avec cette cavité, et dont le rôle sera défini ci-dessus.

Dans les réalisations les plus courantes, les pompes seront logées dans la cassette. Toutefois pour certaines applications ou dans certaines réalisations, les pompes pourront être montées à demeure dans le module permanent.

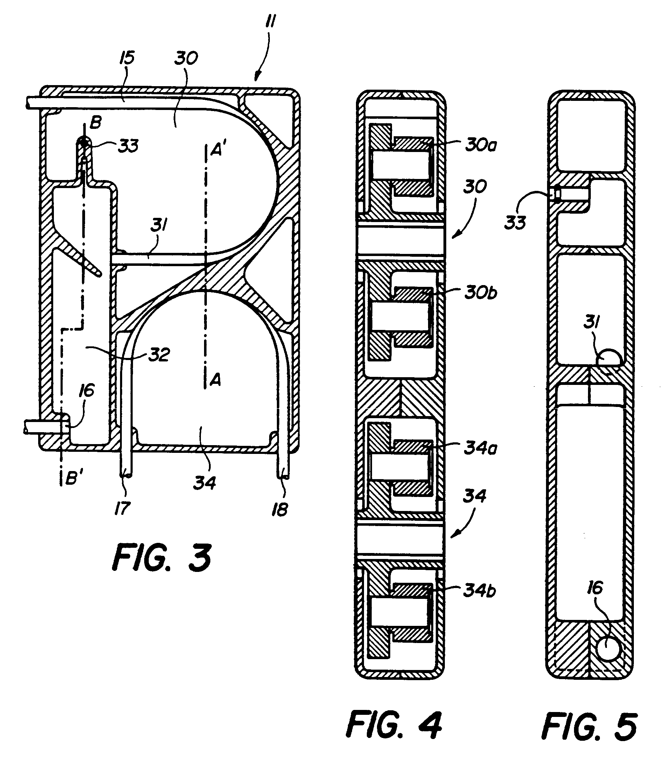

Les figures 3, 4 et 5 illustrent plus en détail une forme de réalisation particulièrement avantageuse d'une cassette 11. Une première pompe péristaltique 30, représentée schématiquement, est associée aux tubulures 15 et 16 dont le rôle a été défini ci-dessus. Dans la forme de réalisation représentée, le conduit de sortie de la pompe péristaltique 30 n'est pas la tubulure 16, mais un conduit 31 qui débouche dans un réservoir 32 qui constitue une réserve de pression. Un raccord de capteur de pression 33 qui communique avec ce réservoir permet de mesurer la pression du liquide au moment où il est transmis dans la zone d'intervention.

Une deuxième pompe péristaltique 34 est associée aux tubulures 17 et 18 dont le rôle a été défini précédemment.

Tous ces éléments sont logés dans un boîtier, de préférence moulé dans une matière synthétique appropriée, qui constitue la cassette proprement dite.

La figure 4 représente une vue en coupe selon la ligne A-A' qui passe par les axes des deux pompes péristaltiques 30 et 34. Cette figure montre les galets 30a et 30 b de la pompe 30 et les galets 34a et 34b de la pompe 34.

La figure 5 représente une vue en coupe de la cassette selon la ligne B-B' et représente notamment le raccord 33 du capteur de pression, l'embouchure du conduit 31 et l'embout de raccordement de la tubulure 16.

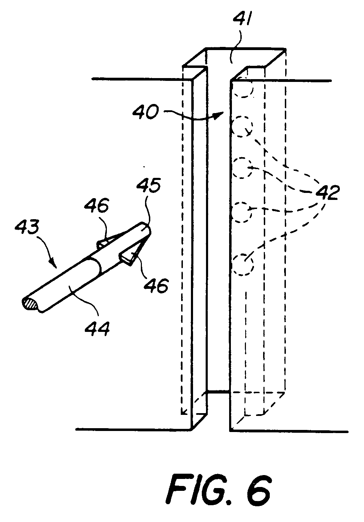

La figure 6 illustre une vue de détail de la rainure 24 qui est une rainure de sélection du programme associé à des moyens de sécurité empêchant une réutilisation d'une cassette. La rainure 24 a un profil particulier et se compose d'une gorge 40 étroite couplée à une gorge élargie 41. Le long de cette gorge sont montés plusieurs contacteurs 42 qui ont la fonction de sélectionner le programme d'utilisation du dispositif en fonction de l'intervention à effectuer. La cassette est munie d'une tige 43 destinée à être engagée dans la rainure 40 dans une position correspondant à l'un des contacteurs 42. De ce fait, la tige 43 se trouvera dans une première position lorsque la cassette est destinée à une intervention urologique, dans une seconde position lorsque l'intervention est du type gynécologique et dans une troisième position pour une arthroscopie, etc. Cette tige 43 se compose d'un téton 44 prolongé par un embout 45 pourvu de deux ailettes latérales 46 rétractables qui peuvent pénétrer à l'intérieur de la gorge large 41 à travers la gorge étroite 40 mais ne peuvent pas en ressortir. De ce fait, lorsque la cassette est retirée à la fin de l'intervention, l'embout 45 est arraché du téton 44 et tombe dans la cavité 23 (voir figure 2). Ce dispositif constitue une sécurité qui empêche la réutilisation d'une cassette.

Il est bien entendu que les contacteurs 42 qui permettent la sélection des programmes en fonction des interventions pourrait être remplacés par tous autres détecteurs d'identification tels que des détecteurs optiques, magnétiques, capacitifs, etc.

Dans le cas d'une arthroscopie du genou, des surpressions importantes peuvent être générées par certains mouvements de ce genou. Le dispositif décrit ci-dessus est équipé d'un asservissement électronique qui permet au chirurgien de travailler en toute sécurité et notamment en sécurité de surpression permettant de limiter les pressions à des valeurs acceptables pour les interventions envisagées. Les surpressions intempestives qui interviennent lors de mouvements brusques du genou agissent en principe sur les sécurités de haute pression en déclenchant les pompes péristaltiques. Le module permanent est équipé d'un dispositif qui limite les surpressions de manière à éviter les déclenchements intempestifs des pompes. La régulation de la pression s'obtient par des commandes appropriées des pompes péristaltiques au cours des interventions.

Le dispositif tel que décrit ci-dessus peut subir différentes modifications et se présenter selon différentes variantes évidentes pour l'homme de l'art. En particulier, il est envisageable de ne prévoir, sur certaines cassettes, qu'une seule pompe péristaltique, lorsque l'intervention correspondante ne justifie pas une aspiration du liquide physiologique et des débris dans la zone d'intervention. D'autre part, la forme du module permanent ainsi que celle des cassettes pourrait être modifiée. Enfin les fonctions des différents composants pourraient également être modifiées afin de tenir compte des exigences spécifiques pour chaque type d'intervention rendue possible avec ce dispositif.

## Revendications

1. Dispositif pour l'irrigation et le drainage continus de tissus ou cavités de l'organisme humain ou animal, comprenant:
- une première pompe péristaltique (30) connectée à une tubulure (15,16) pour fournir un liquide physiologique d'irrigation pendant une intervention endoscopique;
- un module permanent (10) équipé d'un premier moteur à commande d'entraînement de la première pompe péristaltique; et
- au moins une cassette (11) à usage unique amovible équipée de ladite tubulure (15,16);
caractérisé en ce qu'il comporte
- des moyens programmés (43) sur la cassette (11) pour fournir des informations spécifiques au moteur à commande;
- des moyens d'identification (42) sur le module permanent (10) prévues pour être activés par les moyens programmés (43) pour effectuer l'opération de la première pompe péristaltique (30) selon une intervention endoscopique spécifique à effectuer.

2. Dispositif selon la revendication 1, caractérisé en ce qu'il comporte une deuxième pompe péristaltique (34) connectée à une tubulure (17,18) pour effectuer le drainage du liquide physiologique sur commande d'une deuxième moteur d'entraînement.

3. Dispositif selon l'une des revendications 1 ou 2, caractérisé en ce que les pompes péristaltiques (30, 34) sont montées dans le module permanent (10).

4. Dispositif selon l'une des revendications 1 ou 2, caractérisé en ce que les pompes péristaltiques (30, 34) sont montées dans la cassette (11), des axes d'entraînement (20, 21) de ces pompes étant montés sur le module permanent (10).

5. Dispositif selon la revendication 4, caractérisé en ce que la cassette (11) est équipée d'au moins quatre tubulures (15, 16, 17, 18) correspondant par paires à chacune desdites pompes péristaltiques (30, 34) une première paire (15, 16) de tubulures dont l'une (15) est couplée à un réservoir de liquide physiologique et dont l'autre (16) est connectée à un instrument d'intervention sur le patient pour amener ledit liquide physiologique dans la zone d'intervention, et une deuxième paire de tubulures (17, 18) dont l'une (17) est également connectée à un instrument d'intervention pour évacuer le liquide physiologique de la zone d'intervention et dont l'autre (18) est couplée à un circuit d'évacuation.

6. Dispositif selon l'une quelconque des revendications 1 à 5, caractérisé en ce que les moyens programmés comportent au moins un organe de codage (43) déposé à un emplacement déterminé en fonction de l'intervention prévue et en ce que les moyens d'identification comportent une série de détecteurs (42) dont l'un au moins est activé par l'organe de codage (43) de la cassette (11).

7. Dispositif selon la revendication 6, caractérisé en ce que l'organe de codage (43) est un téton (44) protubérant ménagé sur une face de la cassette (11).

8. Dispositif selon la revendication 6, caractérisé en ce que les détecteurs (42) sont des interrupteurs activés mécaniquement.

9. Dispositif selon la revendication 6, caractérisé en ce que les détecteurs (42) sont des détecteurs optiques.

10. Dispositif selon la revendication 6, caractérisé en ce que les détecteur (42) sont des détecteurs magnétiques.

11. Dispositif selon la revendication 6, caractérisé en ce que les détecteurs (42) sont des détecteurs capacitifs.

12. Dispositif selon l'une quelconque des revendications 1 à 11, caractérisé en ce qu'il comporte des moyens de sécurité pour empêcher une réutilisation de la cassette.

13. Dispositif selon la revendication 12, caractérisé en ce que lesdits moyens de sécurité comportent un téton (44) solidaire de la cassette (11), agencé pour s'engager dans une rainure (40, 41) du module permanent (10) et conçu pour se casser lors du retrait de la cassette.

14. Dispositif selon l'une quelconque des revendications 1 à 13, caractérisé en ce que les moyens de sécurité sont associés aux moyens d'identification.

## Claims

1. Device for continuously irrigating and draining tissue or cavities in the organism of humans or animals, comprising:
- a first peristaltic pump (30) connected to a fitting (15, 16) for supplying a physiologic irrigating liquid during an endoscopic intervention;
- a permanent module (10) equipped with a first drive motor for driving said first peristaltic pump; and
- at least one detachable disposable cartridge (11) equipped with said fitting (15, 16),
**characterized in** that it includes
- programmed means (43) on said cartridge (11) for supplying specific information to said drive motor;
- identifying means (42) on said permanent module (10) which are provided for activation by means of said programmed means (43) so as to operate said first peristaltic pump (30) in dependence on a specific endoscopic intervention to be carried out.

2. Device according to Claim 1, **characterized in** that it includes a second peristaltic pump (34) connected to a fitting (17, 18) for evacuating said physiologic liquid in response to a command from a second drive motor.

3. Device according to any of the Claims 1 or 2, **characterized in** that said peristaltic pumps (30, 34) are mounted in said permanent module (10).

4. Device according to any of the Claims 1 or 2, **characterized in** that said peristaltic pumps (30, 34) are disposed in said cartridge (11), with the driving axes (20, 21) of these pumps being mounted on said permanent module (10).

5. Device according to Claim 4, **characterized in** that said cartridge (11) is equipped with at least four fittings (15, 16, 17, 18) which are associated each, in pairs, with one of said peristaltic pumps (30, 34), specifically a first pair of fittings (15, 16), whereof one (15) is attached to a reservoir of said physiologic liquid whilst the other (16) is connected to an instrument for the intervention to be performed on the patient for supplying said physiologic liquid into the area of intervention, and a second pair of fittings (17, 18), whereof one (17) is equally connected to an intervention instrument for evacuating said physiologic liquid from the area of intervention whilst the other (18) is coupled to an evacuation circuit.

6. Device according to any of the Claims 1 to 5, **characterized in** that said programmed means include at least one encoding unit (43) placed at a location determined in dependence on the envisaged intervention, and that said identifying means include a series of detectors (42) whereof at least one is activated by said encoding unit (43) in said cartridge (11).

7. Device according to Claim 6, **characterized in** that said encoding unit (43) is a protruding extension (44) formed on a surface of said cartridge (11).

8. Device according to Claim 6, **characterized in** that said detectors (42) are mechanically operable switches.

9. Device according to Claim 6, **characterized in** that said detectors (42) are optical detectors.

10. Device according to Claim 6, **characterized in** that said detectors (42) are magnetic detectors.

11. Device according to Claim 6, **characterized in** that said detectors (42) are capacitive detectors.

12. Device according to any of the Claims 1 to 11, **characterized in** that it comprises safety means preventing said cartridge from being reused.

13. Device according to Claim 12, **characterized in** that said safety means include an extension (44) fixedly connected to said cartridge (11), which extension is disposed for engagement in a recess (40, 41) on said permanent module (10) and designed for breaking during withdrawal of said cartridge.

14. Device according to any of the Claims 1 to 13, **characterized in** that said safety means are associated with said identifying means.

## Patentansprüche

1. Vorrichtung zum kontinuierlichen Befeuchten und Drainieren von Gewebe oder Hohlräumen im menschlichen oder tierischen Organismus, welche folgendes aufweist:
- eine mit einem Anschlußstutzen (15, 16) verbundene erste Quetschschlauchpumpe (30) zur Zuführung einer physiologischen Befeuchtungsflüssigkeit während eines endoskopischen Eingriffs;
- ein mit einem ersten Antriebsmotor ausgerüstetes ortsfestes Modul (10) für den Antrieb der ersten Quetschschlauchpumpe; und
- mindestens eine abnehmbare Kassette (11) zur Einmalverwendung, die mit dem Anschlußstutzen (15, 16) ausgestattet ist,
**dadurch gekennzeichnet,** daß sie
- programmierte Einrichtungen (43) auf der Kassette (11) aufweist, welche spezielle Informationen an den Antriebsmotor liefert;
- Identifiziereinrichtungen (42) auf dem ortsfesten Modul (10), die so zur Aktivierung mittels der programmierten Einrichtungen (43) vorgesehen sind, daß die erste Quetschschlauchpumpe (30) je nach einem vorzunehmenden speziellen endoskopischen Eingriff betrieben wird.

2. Vorrichtung nach Anspruch 1, **dadurch gekennzeichnet,** daß sie eine zweite Quetschschlauchpumpe (34) aufweist, die an einen Anschlußstutzen (17, 18) zum Absaugen der physiologischen Flüssigkeit auf einen Befehl eines zweiten Antriebsmotors hin angeschlossen ist.

3. Vorrichtung nach einem der Ansprüche 1 oder 2, **dadurch gekennzeichnet,** daß die Quetschschlauchpumpen (30, 34) in dem ortsfesten Modul (10) angeordnet sind.

4. Vorrichtung nach einem der Ansprüche 1 oder 2, **dadurch gekennzeichnet,** daß die Quetschschlauchpumpen (30, 34) in der Kassette (11) angeordnet sind, wobei die Antriebsachsen (20, 21) dieser Pumpen auf dem ortsfesten Modul (10) angebracht sind.

5. Vorrichtung nach Anspruch 4, **dadurch gekennzeichnet,** daß die Kassette (11) mit mindestens vier Anschlußstutzen (15, 16, 17, 18) ausgerüstet ist, welche paarweise jeweils einer der Quetschschlauchpumpen (30, 34) zugeordnet sind, und zwar ein erstes Paar (15, 16) der Anschlußstutzen, wovon einer (15) an einen Vorratsbehälter für die physiologische Flüssigkeit angeschlossen ist und der andere (16) mit einem Instrument für den am Patienten vorzunehmenden Eingriff zur Zuleitung der physiologischen Flüssigkeit in das Operationsgebiet verbunden ist, und ein zweites Paar Anschlußstutzen (17, 18), wovon einer (17) zum Absaugen der physiologischen Flüssigkeit aus dem Operationsgebiet ebenfalls mit einem für den Eingriff vorgesehenen Instrument verbunden ist und der andere (18) mit einem Absaugkreis gekoppelt ist.

6. Vorrichtung nach einem der Ansprüche 1 bis 5, **dadurch gekennzeichnet,** daß die programmierten Einrichtungen mindestens einen Kodierer (43) aufweisen, der an einer Stelle angeordnet ist, die entsprechend dem vorgesehenen Eingriff festgelegt wird, und daß die Identifiziereinrichtungen eine Reihe von Sensoren (42) aufweisen, von denen mindestens einer durch den Kodierer (43) in der Kassette (11) aktiviert wird.

7. Vorrichtung nach Anspruch 6, **dadurch gekennzeichnet,** daß der Kodierer (43) ein vorstehender Ansatz (44) ist, der auf einer Fläche der Kassette (11) ausgebildet ist.

8. Vorrichtung nach Anspruch 6, **dadurch gekennzeichnet,** daß die Sensoren (42) mechanisch betätigte Schalter sind.

9. Vorrichtung nach Anspruch 6, **dadurch gekennzeichnet,** daß die Sensoren (42) optische Sensoren sind.

10. Vorrichtung nach Anspruch 6, **dadurch gekennzeichnet,** daß die Sensoren (42) magnetische Sensoren sind.

11. Vorrichtung nach Anspruch 6, **dadurch gekennzeichnet,** daß die Sensoren (42) kapazitive Sensoren sind.

12. Vorrichtung nach einem der Ansprüche 1 bis 11, **dadurch gekennzeichnet,** daß sie Sicherheitseinrichtungen aufweist, welche eine Wiederverwendung der Kassette verhindern.

13. Vorrichtung nach Anspruch 12, **dadurch gekennzeichnet,** daß die Sicherheitseinrichtungen einen fest mit der Kassette (11) verbundenen Ansatz (44) aufweisen, der so angeordnet ist, daß er in eine Vertiefung (40, 41) auf dem ortsfesten Modul (10) eingreift, und so ausgelegt ist, daß er beim Herausziehen der Kassette zerbricht.

14. Vorrichtung nach einem der Ansprüche 1 bis 13, **dadurch gekennzeichnet,** daß die Sicherheitseinrichtungen den Identifiziereinrichtungen zugeordnet sind.
